# EUROPEAN PATENT SPECIFICATION

(11) **EP 1 460 173 B1**
(45) Date of publication and mention of the grant of the patent: **14.05.2008**
(21) Application number: 04005126.0
(22) Date of filing: 04.03.2004
(51) Int. Cl.: D21F 7/10

(54) **Pin seamed papermaker's press felt with cross machine direction yarns woven in dreher weave at seam loops**
Papiermaschinenpressfilz mit einer Verbindungsnaht und nach Dreher-Webart gewobenen Querfäden bei den Nahtschlaufen
Feutre de presse de machine à papier à jonction comportant des fils transversaux tissés suivant une armature de dreher au niveau des boucles de jonction

(30) Priority: 20.03.2003 US 393085
(43) Date of publication of application: 22.09.2004
(73) Proprietor: WEAVEXX CORPORATION, Wake Forest, NC 27587 (US)
(72) Inventor: Gstrein, Hippolit, 2640 Gloggnitz (AT)
(74) Representative: Popp, Eugen

(56) References cited:
- US-A1- 2002 112 274
- US-B1- 6 267 150

## Description

### Field of the Invention

The present invention relates generally to papermaking, and more particularly to fabrics used in papermaking.

### Background of the Invention

In the conventional fourdrinier papermaking process, a water slurry, or suspension, of cellulosic fibers (known as the paper "stock") is fed onto the top of the upper run of an endless belt of woven wire and/or synthetic material that travels between two or more rollers. The belt, often referred to as a "forming fabric," provides a papermaking surface on the upper surface of its upper run which operates as a filter to separate the cellulosic fibers of the paper stock from the aqueous medium, thereby forming a wet paper web. The aqueous medium drains through mesh openings of the forming fabric, known as drainage holes, by gravity alone or with assistance from one or more suction boxes located on the lower surface (i.e., the "machine side") of the upper run of the fabric.

After leaving the forming section, the paper web is transferred to a press section of the paper machine, in which it is passed through the nips of one or more pairs of pressure rollers covered with another fabric, typically referred to as a "press felt." Pressure from the rollers removes additional moisture from the web; the moisture removal is often enhanced by the presence of a "batt" layer on the press felt. The paper is then conveyed to a drier section for further moisture removal. After drying, the paper is ready for secondary processing and packaging.

Press felts typically include one or more base fabric layers; these can be "flat-woven" and formed after weaving into an endless belt, or can be woven in endless form. Generally, the flat-woven process is preferred, as it is typically less expensive and more versatile than the endless weaving process. Also, in many instances the felt is cut widthwise and reattached to simplify installation on a paper machine, in which case some of the advantages of endless weaving (such as the absence of a seam in the fabric) are lost.

Of course, flat weaving a fabric of a base layer requires that provision be made for joining it into endless belts. Such joints should be constructed in such a manner that they are sufficiently strong to withstand the extreme load, temperature, and wear conditions the press felt experiences, yet do not cause the surface of the press felt above the seam to unduly mark the paper. One popular method of joining the base fabric of a press felt is to form loops with machine direction yams on each end of the base fabric. To form the flat-woven base fabric into an endless belt, the ends of the fabric are placed adjacent to each other, with each of the loops on one end positioned between two loops on the other end in interdigitating fashion. A "pin" (usually formed of a single monofilament or monofilament strands) is then inserted into all of the loops to join the ends. After the batt layer(s) are needled or otherwise attached to the base layer, the batt layer(s) are cut at the seam location, the pin is removed, and the finished press felt is shipped to a paper mill. Once at the paper mill, the press felt can be installed by placing it onto a paper machine, then inserting another (usually more flexible) monofilament pin or pintle into the loops. Examples of this type of seam are described in U.S. Patent Nos. 4, 764,417 and 4,737,241 to Gulya; 4,601,785 to Lilja et al., and 5,476,123 to Rydin, the disclosures of which are hereby incorporated herein by reference in their entireties.

Not unexpectedly, the presence of a pin seam can create heterogeneity in the press felt in the pin seam area. For example, the compressibility and water and air permeability may be different at the seam area compared to the remainder of the press felt. Further, because the density of base fabric yams is lower in the seam area, the batt fibers tend to be anchored less effectively in the seam. One approach to address some of these problems is to weave one or more additional yarns into the pin seam area that increase the yarn density on the paper side of the fabric. Examples of this approach are disclosed in the aforementioned U.S. Patent No. 5,476,123 to Rydin in U.S. Patent Nos. 5,913,339 to Lee and 5,799,709 to Shipley, in U.S. Patent Application US 2002/0112274 and in U.S. Patent No. 6,267,150.

### Summary of the Invention

The present invention is directed to pin-seamed press felts with additional CMD yams located in the pin seam loops. As a first aspect, the invention is directed to a press felt for a papermaking machine, comprising: a base fabric comprising a plurality of machine direction (MD) yarns interwoven with a plurality of cross machine direction (CMD) yams in a predetermined regular weave pattern, the MD yarns being divisible into upper MD yarns, lower MD yams, and seam loops merging with either end of the upper and lower MD yams, the seam loops defining the longitudinal ends of the press felt and having upper and lower portions; and a first Dreher CMD yarn interwoven with the CMD yarn of the regular weave pattern located nearest to the seam loops at one end at the press felt and with the upper portions of the seam loops at said one end of the press felt in a Dreher weave. In this configuration, the press felt can have added yarn density at the seam to improve the uniformity of the press felt at the seam. Also, the use of the Dreher yarn can precisely fix the position of the last CMD yarn of the regular weave.

As a second aspect, the invention is directed to a press felt for a papermaking machine, comprising: a base fabric comprising a plurality of machine direction (MD) yarns interwoven with a plurality of cross machine direction (CMD) yarns in a predetermined regular weave pattern, the MD yarns being divisible into upper MD yarns, lower MD yarns, and seam loops merging with either end of the upper and lower MD yams, the seam loops defining the longitudinal ends of the press felt and having upper and lower portions; an additional CMD yam that passes between the upper and lower portions of each of the seam loops at one end of the press felt; and a first Dreher CMD yarn interwoven with the additional CMD yarn and with the upper portions of the seam loops at said one end of the press felt in a Dreher weave. This configuration can provide the same types of benefits as are mentioned above.

### Brief Description of the Figures

**Figure 1** is a schematic diagram illustrating the press section of a papermaking machine that may employ a press felt according to embodiments of the present invention.
**Figure 2** is an enlarged, partial, cutaway perspective view of the press felt of **Figure 1** showing the pin seam of the press felt of Figure 1.
**Figures 3A** and **3B** are greatly enlarged, partial perspective views of the pin seam of **Figure 2**, with **Figure 3A** showing the pin seam assembled and **Figure 3B** showing the pin seam unassembled.
**Figure 4** is a greatly enlarged, partial perspective view of seam loops and additional yarns according to another embodiment of the present invention.
**Figure 5** is a greatly enlarged, partial perspective view of seam loops and additional yams according of an additional embodiment of the present invention.
**Figure 6** is a greatly enlarged, partial perspective view of seam loops and additional yams according to a further embodiment of the present invention.
**Figure 7** is a greatly enlarged, partial perspective view of seam loops and additional yarns according to still another embodiment of the present invention.
**Figure 8** is a greatly enlarged, partial perspective view of seam loops and additional yams according to yet another embodiment of the present invention.
**Figure 9** is a greatly enlarged, partial perspective view of seam loops and additional yams according to even another embodiment of the present invention.
**Figure 10** is a greatly enlarged, partial perspective view of seam loops and additional yarns according to still another embodiment of the present invention.

### Detailed Description of Embodiments of the Present Invention

The present invention will now be described more fully hereinafter, in which embodiments of the invention are shown. This invention may, however, be embodied in different forms and should not be construed as limited to the embodiments set forth herein. Rather, these embodiments are provided so that this disclosure will be thorough and complete, and will fully convey the scope of the invention to those skilled in the art. In the drawings, like numbers refer to like elements throughout. Thicknesses and dimensions of some components may be exaggerated for clarity.

As used herein, the terms "machine direction" (MD) and "cross machine direction" (CMD) refer, respectively, to a direction aligned with the direction of travel of the papermakers' fabric on a papermaking machine, and a direction parallel to the fabric surface and transverse to the direction of travel. Also, both the flat weaving and endless weaving methods described hereinabove are well known in the art, and the term "endless belt" as used herein refers to belts made by either method.

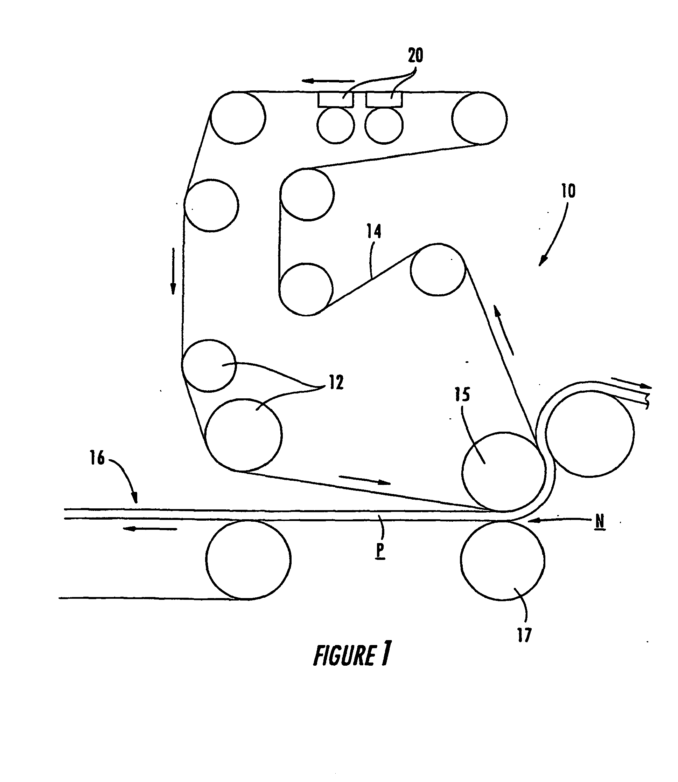

Referring now to the drawings, a papermaking machine press section, designated broadly at **10**, is illustrated in **Figure 1**. The press section **10** includes a press felt **14** that is installed upon and conveyed by a set of rollers **12**. In its travel, the felt **14** travels over a press roll **15**. An opposed press roll **17** is positioned so that, in conjunction with the felt **14** and press roll **15**, it forms a nip **N** between the press rolls **15**.

In operation, a paper web **P** is conveyed from a forming section **16** through the nip **N** formed by the press rollers **15, 17,** wherein pressure is applied to the paper web **P** by the press rolls **15, 17.** The pressure forces moisture from the paper web **P** that is absorbed by the felt **14.** As the felt **14** is conveyed around its roller set **12**, moisture is removed therefrom, and the felt **14** is conditioned by one or more suction boxes **20**.

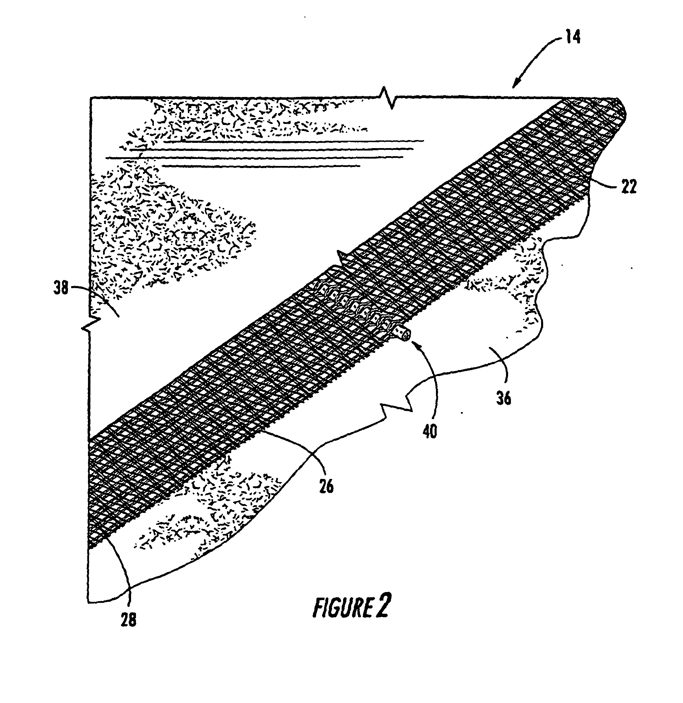

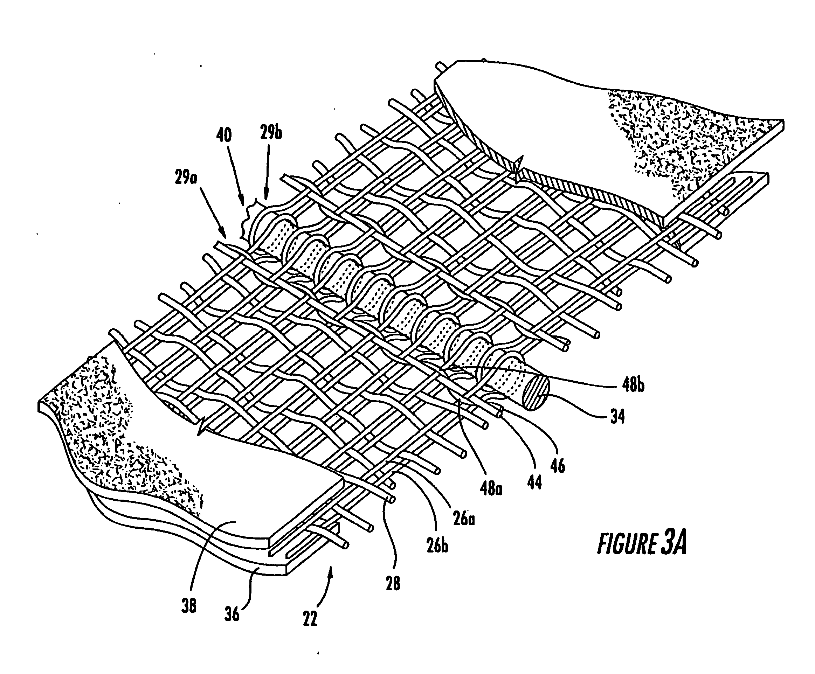

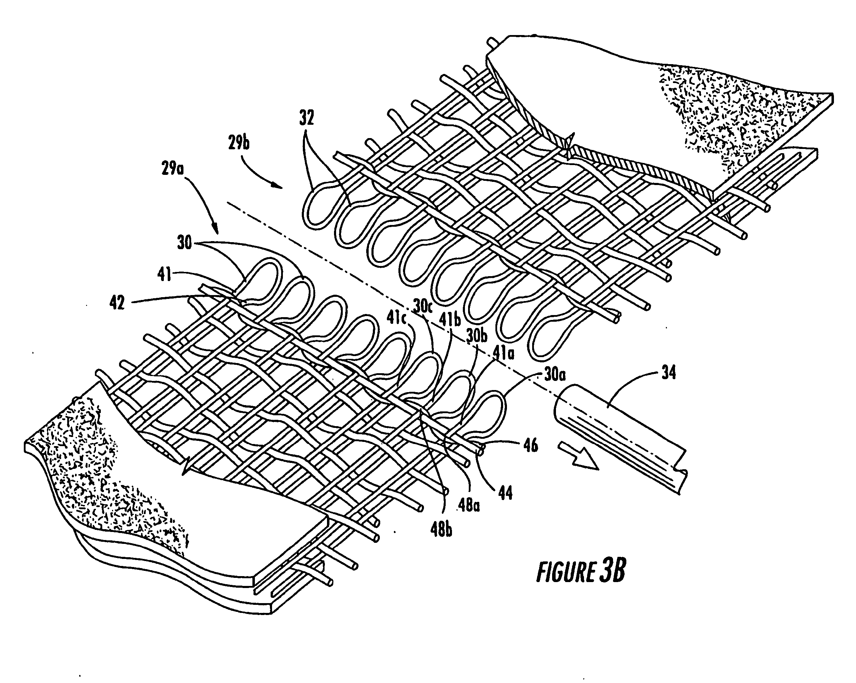

**Figures 2****,** **3A** and **3B** illustrate enlarged sections of the felt **14**. As can be seen in the aforementioned figures, the felt **14** includes a base fabric layer **22** which includes a set of machine direction yarns **26** (divisible into upper MD yams **26a** and lower MD yams **26b**) and a set of cross machine direction yarns **28** interwoven in a regular pattern with the machine direction yarns **26**. The CMD yarns **28** are interwoven with the MD yams **26** such that each CMD yam **28** passes, sequentially, over one upper MD yam **26a**, between the next pair of upper and lower MD yarns **26a**, **26b**, below the next lower MD yarn **26b**, and between the next pair of upper and lower MD yarns **26a**, **26b**. Adjacent CMD yarns **28** are offset from one another by one or more MD yarns **26** yams to form, in the illustrated embodiment, a 1x4 satin pattern on the top surface of the base fabric **22**.

The base fabric **22** is woven in a flat weave process; thus, in a flat condition, the fabric **22** has two free ends **29a**, **29b**, one of which (**29a**) includes seam loops **30** (formed by MD yarns **26**), and the other of which (**29b**) includes seam loops **32** formed by the other ends of the MD yarns **26**. When the base fabric **22** is in an endless condition such as that illustrated in **Figures 2** and **3A**, the loops **30**, **32** are positioned in interdigitated fashion, and a pin **34** is inserted through the loops **30**, **32** to join the ends **29a**, **29b** of the base fabric **22** to form a seam **40**. It can be seen that the seam loops **30** include upper portions **41** and lower portions **42**.

Those skilled in this art will recognize that other types of fabrics can be employed as the lower fabric layer of the base fabric layer **22** of the press felt **14**, including other single layer fabrics, duplex fabrics (those having two sets of MD yarns and one set of CMD yarns), and triplex fabrics (i.e., those having two sets of machine direction yarns and two sets of cross machine direction yarns). Virtually any weave pattern known to those skilled in this art, such as the illustrated plain weave, twills, satins, and the like, can be used for this fabric layer.

Referring now to **Figures 3A** and **3B**, at the seam **40**, the upper portions **41** of the seam loops **30** are interwoven with two additional yams: an anchoring yarn **44** and a Dreher yam **46**. The anchoring yam **44** passes between the upper portions **41** and the lower portions **42** of each of the seam loops **30**. The Dreher yarn **46** interweaves with the anchoring yam **44** and the upper portions **41** of the seam loops **30** in a Dreher weave, which is described in detail below.

A Dreher weave is one in which a first yarn weaves on both sides of a second yam that extends in nominally the same direction (*i.e.,* both the first and second yarns are MD yams or both are CMD yams), wherein the first yarn passes below the second yarn and passes over and interlaces with multiple yarns that extend nominally in the opposite direction. Consequently, the second yam of the Dreher weave passes above the first yarn and interlaces with the oppositely extending yams by passing below them. The result is a somewhat serpentine path followed by at least one of the yarns. Additional information regarding Dreher weaves is set forth in, for example, *Gewebetechnik,* (VEB - Fachbuchverlag Leipzig, 1978).

A Dreher weave is demonstrated in the base fabric **22** shown in **Figure 3B** by the interweaving of the Dreher yam **46** and the anchoring yam **44** with the upper portions **41** of the seam loops **30**. As can be seen in **Figure 3B**, the Dreher yam **46** passes over the upper portion **41a** of the seam loop **30a** on the side of the anchoring yarn **44** away from the seam **40**, under the anchoring yarn **44**, above the upper portion **41b** of the next seam loop **30b** on the side of the anchoring yarn **44** adjacent the seam **40,** under the anchoring yarn **44,** over the upper portion **41c** of the seam loop **30c**, and so on in a repeating pattern. Thus, in the illustrated embodiment the Dreher yarn **46** forms single float knuckles **48a**, **48b** over each of the seam loop upper portions **41**, wherein the knuckles are alternately positioned on opposite sides of the anchoring yarn **44** as the Dreher yam **46** extends in the cross machine direction.

In this configuration, the anchoring yarn **44** is positioned to provide a yam at the seam **40** that can help to increase the yarn density at the seam **40** and that can assist in anchoring batt fibers inserted in the seam area. Also, the Dreher yam can assist in precisely fixing the position of the anchoring yarn **44** in the seam area.

Typically, the anchoring yam **44** may be of any form (**e.g.**, monofilament, multifilament, hybrid yarns, meltable monofilaments, and twists) known to be suitable for use in press felts, although monofilaments or twists are preferred. The anchoring yam will typically have a diameter of between about 0.2 and 0.5 mm or, if in three ply twists, each monofilament will typically have a diameter of between about 0.1 and 0.3 mm each. The Dreher yarn may take any form (*e.g.*, monofilament, multifilament, hybrid yams, meltable monofilaments, and twists), with multifilaments being preferred. The Dreher yam will typically have a fineness of between about 50 and 500 dtex. These desciptions of the Dreher yarn and the anchoring yam are equally applicable to the press felt embodiments described below.

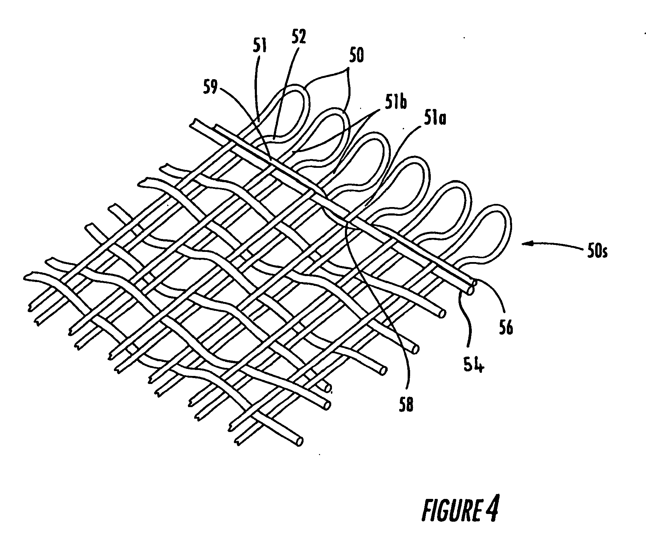

The concept of employing a Dreher weave at the seam loops can be extended to other configurations. For example, another embodiment of a press felt having seam loops **50** that form a seam **50**s is illustrated in **Figure 4**. In this embodiment, an anchoring yarn **54** passes between upper portions **51** and lower portions **52** of the seam loops **50** in the manner described with respect to the embodiment of **Figures 3A** and **3B**. However, a Dreher yarn **56** is woven such that it forms single float knuckles **58** over the upper portions **51a** on one side of the anchoring yarn **54** (the side away from the seam **50s**) and three yarn floats **59** (*i.e.*, the Dreher yam **54** passes over the upper portions **51b** of the seam loops **50** of three consecutive MD yarns) on the opposite side of the anchoring yarn **54**. As with the embodiment of **Figures 3A** and **3B**, the Dreher yarn **56** can help to precisely fix the position of the anchoring yam **54**, and the anchoring yam **54** can increase the density at the seam and provide anchoring for batt fibers.

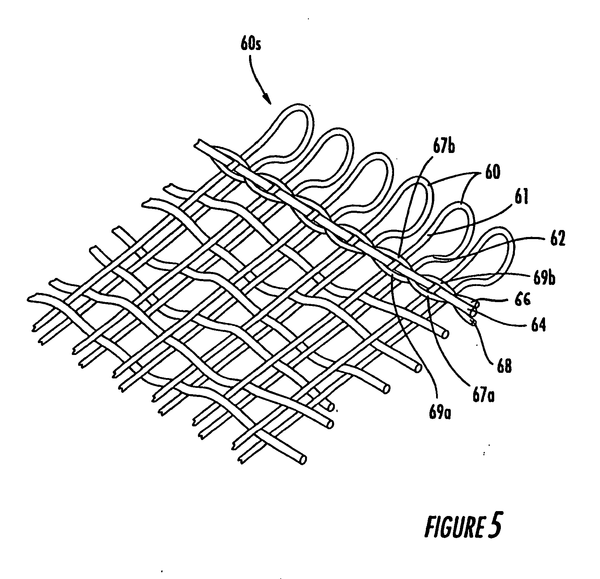

An additional embodiment of a press felt of the invention is illustrated in **Figure 5**. The press felt illustrated therein includes seam loops **60** with upper portions **61** and lower portions **62** as well as an anchoring yarn **64** that passes between the upper and lower portions **61**, **62** of the seam loops **60**. One Dreher yam **66** alternately forms knuckles **67a**, **67b** on opposite sides of the anchoring yarn **64** as it passes over the upper portions **61** of the seam loops **60**. A second Dreher yam **68** is woven such that it forms knuckles **69a** over the upper portions **61** away from the seam **60s** when the Dreher yarn **66** is forming knuckles **67b** adjacent the seam **60s**, and forms knuckles **69b** over upper portions **61** of the seam loops **60** adjacent the seam **60s** when the Dreher yarn **66** is forming knuckles **67a** away from the seam **60s** (*i.e*., the first and second Dreher yams are woven in opposite Dreher patterns). The form, material and size of the second Dreher yarn may be the same as what is described above for the first Dreher yarn, or it may differ as warranted.

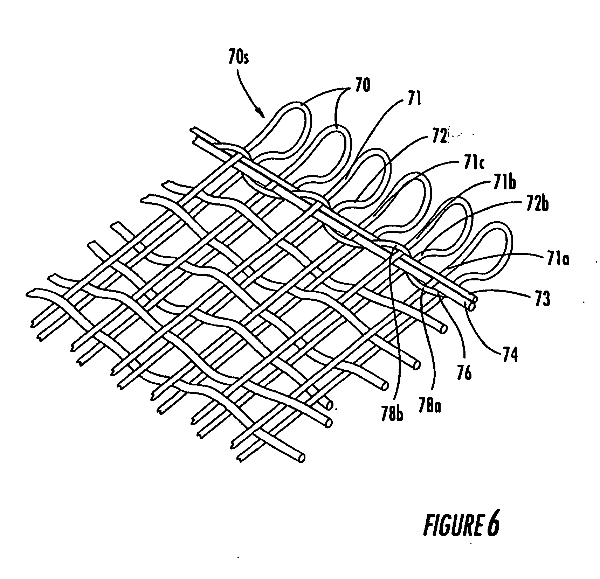

A further embodiment of a press felt of the present invention is illustrated in **Figure 6**. The press felt illustrated therein includes seam loops **70** having upper portions **71** and lower portions **72** and an anchoring yam **74** that passes between the upper and lower portions **71**, **72**. An additional CMD yam **73** is woven with the upper portions **71** of the seam loops **70** nearer to the seam **70s** than the anchoring yarn **74**. The additional CMD yarn **73** alternately passes over the upper portion **71a** of one MD yarn, then between the upper and lower portions **71b**, **72b** of the next MD yarn **71**, then above the upper portion **71c** of the next MD yarn **71**, and so on in a repeating pattern. A Dreher yarn **76** forms single float knuckles **78a**, **78b** over alternate seam loop upper portions **71** on opposite sides of both the additional CMD yarn **73** and the anchoring yarn **74** and passes below both the additional CMD yarn **73** and the anchoring yarn **74** as it extends between adjacent MD yarns to form the aforementioned knuckles **78a, 78b**.

The additional CMD yarn **73** typically takes the form of a single ply or mutli-ply yam, but may take any form. The diameter of the additional CMD yarn is typically between about 0.2 mm and 0.5 mm, or it may have a fineness of between about 50 and 500 dtex. This description is equally applicable to additional CMD yams described below in other embodiments.

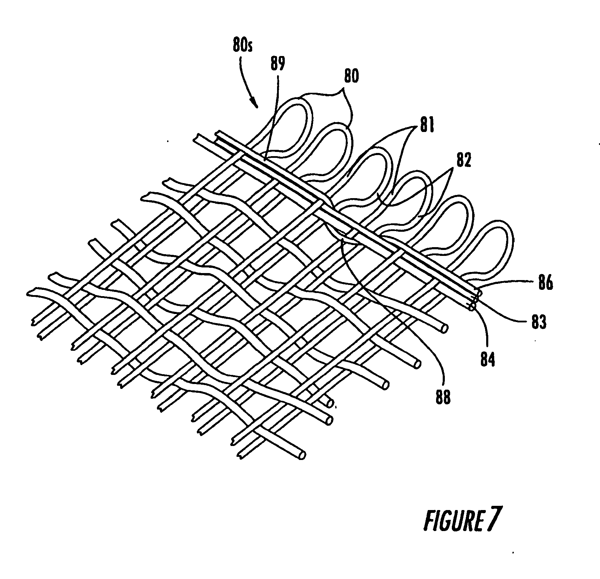

Another embodiment of a press felt of the invention is illustrated in **Figure 7**. The press felt shown in **Figure 7** includes seam loops **80** with upper and lower portions **81**, **82**, and includes an additional CMD yarn **83** and an anchoring yarn **84** like those described above in connection with the embodiment of **Figure 6**. A Dreher yam **86** forms, alternately, single float knuckles **88** on the side of the additional CMD yarn **83** and the anchoring yarn **84** away from the seam **80s** and three-yarn floats **89** over the seam loop upper portions **81** near the seam **80s**.

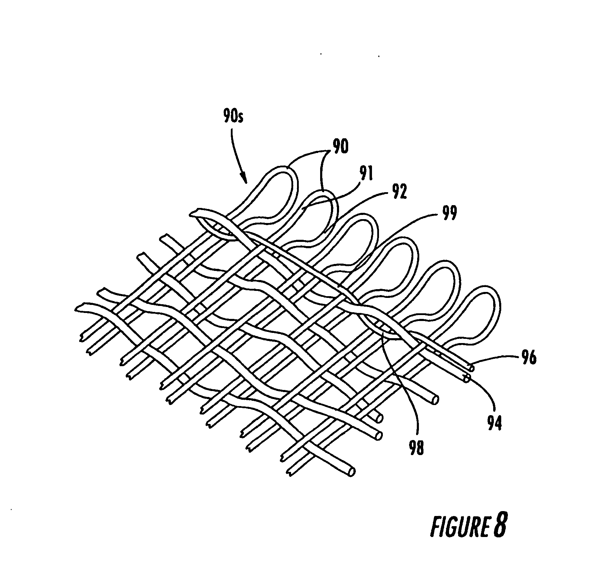

An additional embodiment of a press felt of the present invention is illustrated in **Figure 8**. In this embodiment, the press felt includes seam loops **90** with upper and lower portions **91**, **92**. Rather than employing an anchoring yarn like those described above and illustrated in **Figures 3A-7**, in this embodiment a Dreher yarn **96** interweaves in a Dreher pattern with the CMD yam **94** of the fabric that follows the regular weave pattern and that is nearest to the seam loops **90**. The Dreher yarn **96** interweaves to form single float knuckles **98** on the side of the CMD yarn **94** away from the seam **90s** and three-yarn floats **99** on the side of the CMD yarn **94** nearest the seam **90s**.

Typically, the CMD yam **94** nearest the seam **90s** will take the same form, will be formed of the same material, and will have the same diameter as the other CMD yarns of the press felt. However, this need not be the case, and the CMD yarn **94** may take other forms known to those skilled in this art to be suitable for use in press felts. This description is equally applicable to the embodiments below in which the CMD yarn nearest the seam is interwoven with a Dreher yam.

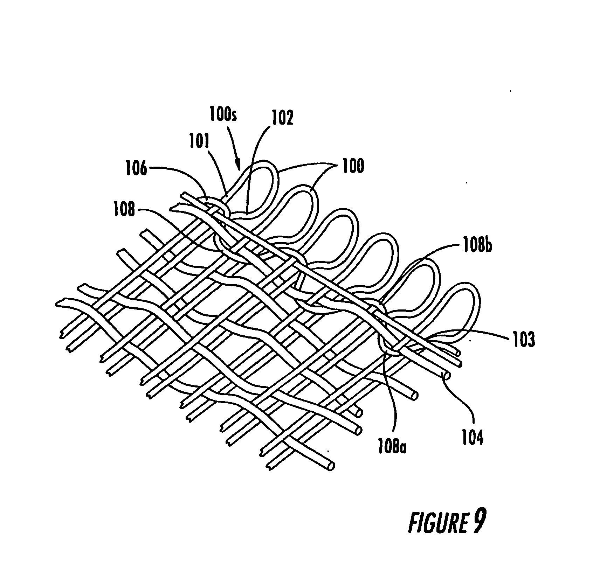

Referring now to **Figure 9**, another embodiment of a press felt of the present invention is illustrated therein. This embodiment includes seam loops **100** with upper portions **101** and lower portions **102**. A CMD yam **104** is the CMD yarn of the regular weave pattern that is positioned nearest to the seam **100s**. An additional CMD yam **103** interweaves in an "over/under/over/under" sequence with the seam loop upper portions **101**. A Dreher yarn **106** passes below the CMD yam **104** and the additional CMD yarn **103** as it interweaves to form knuckles **108a**, **108b** over alternate seam loop upper portions **101** of alternate MD yarns on opposite sides of the CMD yam **104** and the additional yarn **103** in the manner described above for the embodiments of **Figures 3A**, **5** and **6**.

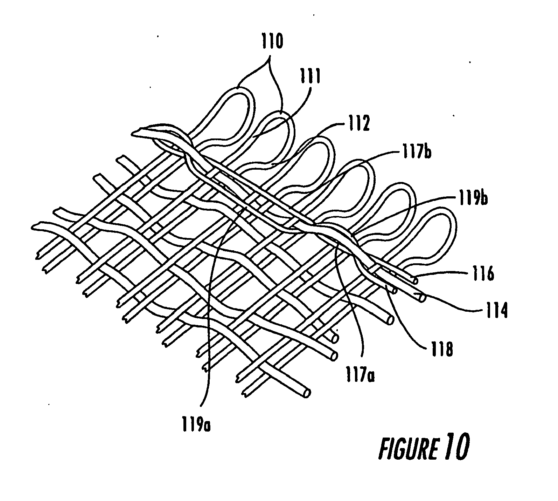

Referring now to **Figure 10**, a still further embodiment of a press felt of the invention is illustrated. This embodiment includes seam loops **110** with upper portions **111** and lower portions **112**. A CMD yam **114** is the CMD yarn of the regular weave pattern that is positioned nearest the seam. A Dreher yarn **116** is interwoven with the CMD yarn **114** in a Dreher weave to form knuckles **117a** on the side of the CMD yarn away from the seam **110s** and three-yarn floats **117b** on the side of the CMD yarn nearest the seam **110s**. A second Dreher yarn **118** is woven in a Dreher weave with the CMD yarn **114** oppositely from the Dreher yarn **116**, such that it forms knuckles **119b** on the side of the CMD yarn **114** nearest the seam **110s** and three-float knuckles **119a** on the side of the CMD yarn **114** away from the seam **110s**.

Those skilled in this art will recognize that other combinations of Dreher weaves at the seam loop are also possible. For example, a press felt may include an anchoring yam of the type shown in the embodiments of **Figures 3A-7** in combination with the Dreher yam patterns shown in **Figure 10**. As another example, a press felt may include the nearest CMD yarn of the regular pattern shown in **Figure 10** with two Dreher yarns woven as shown in **Figure 5**, such that each Dreher yarn forms knuckles on both sides of that CMD yarn. Other modifications which can provide similar benefits and performance advantages may be apparent to those skilled in this art and need not be described in detail herein.

For any of the illustrated or described embodiments, the press felt of the invention may also include one or more batt layers. Referring back to **Figure 2**, the press felt **14** includes two batt layers: a machine side batt layer **120** and a paper side batt layer **122**. Illustratively and preferably, these batt layers **120**, **122** are attached to the base fabric layer **22** through a needling process, although other attachment techniques, such as heat bonding and adhesives, can also be used with the present invention. The machine side and paper side batt layers **120**, **122** should be formed of material, such as a synthetic fiber like acrylic, aramid, polyester, or nylon, or a natural fiber such as wool, that assists in wicking water away from the base fabric layer **22**. Preferred materials for the batt layers **120, 122** include polyamide, polyester and blends thereof. The weight and thickness of the batt layers **120, 122** can vary, although it is preferably that the ratio of batt weight to fabric weight is about between about 0.5 and 2.0, with 1.0 being more preferred. Also, in some embodiments, it may be desirable to have additional batt layers or to omit either or both of the batt layers **120**, **122**.

The foregoing is illustrative of the present invention and is not to be construed as limiting thereof. Although exemplary embodiments of this invention have been described, those skilled in the art will readily appreciate that many modifications are possible in the exemplary embodiments without materially departing from the novel teachings and advantages of this invention. Accordingly, all such modifications are intended to be included within the scope of this invention as recited in the claims. The invention is defined by the following claims.

## Claims

1. A press felt for a papermaking machine, comprising:
a base fabric comprising a plurality of machine direction (MD) yarns interwoven with a plurality of cross machine direction (CMD) yarns in a predetermined regular weave pattern, the MD yarns being divisible into upper MD yarns, lower MD yarns, and seam loops (90; 100; 110) merging with either end of the upper and lower MD yarns, the seam loops defining the longitudinal ends of the press felt and having upper and lower portions (91, 92; 101, 102; 111, 112); and
a first Dreher CMD yarn (96; 106; 116) interwoven with the CMD yarn (94; 104; 114) of the regular weave pattern located nearest to the seam loops at one end of the press felt and with the upper portions of the seam loops at said one end of the press felt in a Dreher weave.

2. The press felt defined in Claim 1, wherein the first Dreher CMD yam passes over each upper portion of each seam loop.

3. The press felt defined in Claim 2, wherein the first Dreher CMD yam forms a knuckle (108a, 108b) over each upper portion of each seam loop.

4. The press felt defined in Claim 3, further comprising an additional CMD yarn (103) that passes alternately above and below the upper portions of adjacent seam loops.

5. The press felt defined in Claim 2, wherein the first Dreher CMD yarn alternately forms knuckles (98) and floats (99) over upper portions of adjacent seam loops.

6. The press felt defined in Claim 5, wherein the floats formed by the first Dreher CMD yarn are floats formed over three adjacent yarns.

7. The press felt defined in Claim 1, further comprising a second Dreher CMD yarn (118) woven with the upper portions of the seam loops in a Dreher weave that is opposite to the Dreher weave of the first Dreher CMD yarn (116).

8. A press felt for a papermaking machine, comprising:
a base fabric comprising a plurality of machine direction (MD) yarns (26) interwoven with a plurality of cross machine direction (CMD) yarns (28) in a predetermined regular weave pattern, the MD yarns being divisible into upper MD yarns (26a), lower MD yarns (26b), and seam loops (30; 50; 60; 70; 80) merging with either end of the upper and lower MD yarns, the seam loops defining the longitudinal ends of the press felt and having upper and lower portions (41,42;51,52;61,62;71,72;81,82);
an additional CMD yarn (44;54;64;74;84) that passes between the upper and lower portions of each of the seam loops at one end of the press felt ; and
a first Dreher CMD yarn (46;56;66;76;86) interwoven with the additional CMD yam and with the upper portions of the seam loops at said one end of the press felt in a Dreher weave.

9. The press felt defined in Claim 8, wherein the first Dreher CMD yam passes over each upper portion of each seam loop.

10. The press felt defined in Claim 9, wherein the first Dreher CMD yam forms a knuckle (48a, 48b; 67a, 67b; 78a, 78b) over each upper portion of each seam loop.

11. The press felt defined in Claim 9, wherein the first Dreher CMD yarn alternately forms knuckles (58; 88) and floats (59; 89) over upper portions of adjacent seam loops.

12. The press felt defined in Claim 9, further comprising an additional CMD yarn (73;83) that passes alternately above and below the upper portions of adjacent seam loops.

13. The press felt defined in Claim 10, further comprising an additional CMD yarn (73) that passes alternately above and below the upper portions of adjacent seam loops.

14. The press felt defined in Claim 10, further comprising a second Dreher CMD yarn (68) woven with the upper portions of the seam loops in a Dreher weave that is opposite to the Dreher weave of the first Dreher CMD yarn (66).

15. The press felt defined in Claim 11, further comprising a second Dreher CMD yarn woven with the upper portions of the seam loops in a Dreher weave that is opposite to the Dreher weave of the first Dreher CMD yarn.

## Patentansprüche

1. Pressenfilz für eine Papiermaschine, der Folgendes aufweist:
ein Grundgewebe, das eine Vielzahl von Maschinenrichtungsfäden (MR-Fäden) aufweist, die mit einer Vielzahl von Maschinenquerrichtungsfäden (MQR-Fäden) in einem vorbestimmten regelmäßigen Bindungsmuster verwebt sind, wobei die MR-Fäden in obere MR-Fäden, untere MR-Fäden und Nahtschlingen (90; 100; 110) unterteilbar sind, die sich mit jedem Ende der oberen und unteren MR-Fäden vereinigen, wobei die Nahtschlingen die Längsenden des Pressenfilzes definieren und obere und untere Bereiche (91, 92; 101, 102; 111, 112) haben, und
einen ersten MQR-Dreherfaden (96; 106; 116), der mit dem MQR-Faden (94; 104; 114) des regelmäßigen Bindungsmusters, der den Nahtschlingen an einem Ende des Pressenfilzes am nächsten liegt, und mit den oberen Bereichen der Nahtschlingen an dem genannten einen Ende des Pressenfilzes in einer Dreherbindung verwebt ist.

2. Pressenfilz nach Anspruch 1, wobei der erste MQR-Dreherfaden über jeden oberen Bereich jeder Nahtschlinge läuft.

3. Pressenfilz nach Anspruch 2, wobei der erste MQR-Dreherfaden über jedem oberen Bereich jeder Nahtschlinge einen Höcker (108a, 108b) bildet.

4. Pressenfilz nach Anspruch 3, der ferner einen zusätzlichen MQR-Faden (103) aufweist, der alternierend über und unter die oberen Bereiche von benachbarten Nahtschlingen läuft.

5. Pressenfilz nach Anspruch 2, wobei der erste MQR-Dreherfaden über oberen Bereichen von benachbarten Nahtschlingen alternierend Höcker (98) und Flottierfäden (99) bildet.

6. Pressenfilz nach Anspruch 5, wobei die von dem ersten MQR-Dreherfaden gebildeten Flottierfäden Flottierfäden sind, die über drei benachbarten Fäden gebildet sind.

7. Pressenfilz nach Anspruch 1, der ferner einen zweiten MQR-Dreherfaden (118) aufweist, der mit den oberen Bereichen der Nahtschlingen in einer Dreherbindung verwebt ist, die zu der Dreherbindung des ersten MQR-Dreherfadens (116) entgegengesetzt ist.

8. Pressenfilz für eine Papiermaschine, der Folgendes aufweist:
ein Grundgewebe, das eine Vielzahl von Maschinenrichtungsfäden (MR-Fäden) (26) aufweist, die mit einer Vielzahl von Maschinenquerrichtungsfäden (MQR-Fäden) (28) in einem vorbestimmten regelmäßigen Bindungsmuster verwebt sind, wobei die MR-Fäden in obere MR-Fäden (26a), untere MR-Fäden (26b) und Nahtschlingen (30; 50; 60; 70; 80) unterteilbar sind, die sich mit jedem Ende der oberen und unteren MR-Fäden vereinigen, wobei die Nahtschlingen die Längsenden des Pressenfilzes definieren und obere und untere Bereiche (41, 42; 51, 52; 61, 62; 71, 72; 81, 82) haben;
einen zusätzlichen MQR-Faden (44; 54; 64; 74; 84), der zwischen den oberen und unteren Bereichen jeder der Nahtschlingen an einem Ende des Pressenfilzes läuft; und
einen ersten MQR-Dreherfaden (46; 56; 66; 76; 86), der mit dem zusätzlichen MQR-Faden und mit den oberen Bereichen der Nahtschlingen an dem genannten einen Ende des Pressenfilzes in einer Dreherbindung verwebt ist.

9. Pressenfilz nach Anspruch 8, wobei der erste MQR-Dreherfaden über jeden oberen Bereich jeder Nahtschlinge läuft.

10. Pressenfilz nach Anspruch 9, wobei der erste MQR-Dreherfaden über jedem oberen Bereich jeder Nahtschlinge einen Höcker (48a, 48b; 67a, 67b; 78a, 78b) bildet.

11. Pressenfilz nach Anspruch 9, wobei der erste MQR-Dreherfaden über oberen Bereichen von benachbarten Nahtschlingen alternierend Höcker (58; 88) und Flottierfäden (59; 89) bildet.

12. Pressenfilz nach Anspruch 9, der ferner einen zusätzlichen MIQR-Faden (73; 83) aufweist, der alternierend über und unter die oberen Bereiche von benachbarten Nahtschlingen läuft.

13. Pressenfilz nach Anspruch 10, der ferner einen zusätzlichen MQR-Faden (73) aufweist, der alternierend über und unter die oberen Bereiche von benachbarten Nahtschlingen läuft.

14. Pressenfilz nach Anspruch 10, der ferner einen zweiten MQR-Dreherfaden (68) aufweist, der mit den oberen Bereichen der Nahtschlingen in einer Dreherbindung verwebt ist, die zu der Dreherbindung des ersten MQR-Dreherfadens (66) entgegengesetzt ist.

15. Pressenfilz nach Anspruch 11, der ferner einen zweiten MQR-Dreherfaden aufweist, der mit den oberen Bereichen der Nahtschlingen in einer Dreherbindung verwebt ist, die zu der Dreherbindung des ersten MQR-Dreherfadens entgegengesetzt ist.

## Revendications

1. Feutre de presse pour une machine à papier, comportant :
une toile de base comportant une pluralité de fils dans la direction de la machine (MD) entrelacés avec une pluralité de fils dans la direction transversale à la machine (CMD) selon un motif de tissage régulier prédéterminé, les fils MD pouvant être divisés en fils MD supérieurs, fils MD inférieurs et boucles de jonction (90 ; 100 ; 110) se fondant avec l'une ou l'autre extrémité des fils MD supérieurs et inférieurs, les boucles de jonction définissant les extrémités longitudinales du feutre de presse et ayant des parties supérieure et inférieure (91, 92 ; 101, 102 ; 111, 112), et
un premier fil CMD de Dreher (96 ; 106 ; 116) entrelacé avec le fil CMD (94 ; 104 ; 114) du motif de tissage régulier situé le plus proche des boucles de jonction au niveau d'une première extrémité du feutre de presse, et avec les parties supérieures des boucles de jonction au niveau de ladite première extrémité du feutre de presse selon un tissage de Dreher.

2. Feutre de presse selon la revendication 1, dans lequel le premier fil CMD de Dreher passe au-dessus de chaque partie supérieure de chaque boucle de jonction.

3. Feutre de presse selon la revendication 2, dans lequel le premier fil CMD de Dreher forme un croisement (108a, 108b) au-dessus de chaque partie supérieure de chaque boucle de jonction.

4. Feutre de presse selon la revendication 3, comportant en outre un fil CMD supplémentaire (103) qui passe en alternance au-dessus et au-dessous des parties supérieures des boucles de jonction adjacentes.

5. Feutre de presse selon la revendication 2, dans lequel le premier fil CMD de Dreher forme en alternance des croisements (98) et des flottés (99) au-dessus de parties supérieures de boucles de jonction adjacentes.

6. Feutre de presse selon la revendication 5, dans lequel les flottés formés par le premier fil CMD de Dreher sont des flottés formés au-dessus de trois fils adjacents.

7. Feutre de presse selon la revendication 1, comportant en outre un second fil CMD de Dreher (118) tissé avec les parties supérieures des boucles de jonction selon un tissage de Dreher qui est opposé au tissage de Dreher du premier fil CMD de Dreher (116).

8. Feutre de presse pour une machine à papier, comportant:
une toile de base comportant une pluralité de fils dans la direction de la machine (MD) (26) entrelacés avec une pluralité de fils dans la direction transversale à la machine (CMD) (28) selon un motif de tissage régulier prédéterminé, les fils MD pouvant être divisés en fils MD supérieurs (26a), fils MD inférieurs (26b) et boucles de jonction (30 ; 50 ; 60 ; 70 ; 80) se fondant avec l'une ou l'autre extrémité des fils MD supérieurs et inférieurs, les boucles de jonction définissant les extrémités longitudinales du feutre de presse et ayant des parties supérieure et inférieure (41, 42 ; 51, 52 ; 61, 62 ; 71, 72 ; 81, 82),
un fil CMD supplémentaire (44 ; 54 ; 64 ; 74 ; 84) qui passe entre les parties supérieure et inférieure de chacune des boucles de jonction à une première extrémité du feutre de presse, et
un premier fil CMD de Dreher (46 ; 56 ; 66 ; 76 ; 86) entrelacé avec le fil CMD supplémentaire et avec les parties supérieures des boucles de jonction à au moins une extrémité du feutre de presse selon un tissage de Dreher.

9. Feutre de presse selon la revendication 8, dans lequel le premier fil CMD de Dreher passe au-dessus de chaque partie supérieure de chaque boucle de jonction.

10. Feutre de presse selon la revendication 9, dans lequel le premier filtre CMD de Dreher forme un croisement (48a, 48b ; 67a, 67b ; 78a, 78b) au-dessus de chaque partie supérieure de chaque boucle de jonction.

11. Feutre de presse selon la revendication 9, dans lequel le premier fil CMD de Dreher forme en alternance des croisements (58 ; 88) et des flottées (59 ; 89) au-dessus de parties supérieures de boucles de jonction adjacentes.

12. Feutre de presse selon la revendication 9, comportant en outre un fil CMD supplémentaire (73 ; 83) qui passe de manière alternée au-dessus et au-dessous des parties supérieures de boucles de jonction adjacentes.

13. Feutre de presse selon la revendication 10, comportant en outre un fil CMD supplémentaire (73) qui passe de manière alternée au-dessus et au-dessous des parties supérieures de boucles de jonction adjacentes.

14. Feutre de presse selon la revendication 10, comportant en outre un second fil CMD de Dreher (68) tissé avec les parties supérieures des boucles de jonction selon un tissage de Dreher qui est opposé au tissage de Dreher du premier fil CMD de Dreher (66).

15. Feutre de presse selon la revendication 11, comportant en outre un second fil CMD de Dreher tissé avec les parties supérieures des boucles de jonction selon un tissage de Dreher qui est opposé au tissage de Dreher du premier fil CMD de Dreher.
